# EUROPÄISCHE PATENTSCHRIFT

(11) **EP 1 083 162 B1**
(45) Veröffentlichungstag und Bekanntmachung des Hinweises auf die Patenterteilung: **19.12.2001**
(21) Anmeldenummer: 00114998.8
(22) Anmeldetag: 21.07.2000
(51) Int. Cl.: C07C 45/54, C07C 49/14

(54) **Verfahren zur Herstellung von 1,3-Diketonen**
Process for the preparation of 1,3-diketones
Procédé pour la préparation de 1,3-dicétones

(30) Priorität: 13.08.1999 DE 19938341
(43) Veröffentlichungstag der Anmeldung: 14.03.2001
(73) Patentinhaber: Wacker-Chemie GmbH, 81737 München (DE)
(72) Erfinder: Dempf, Dominik, Dr., 84489 Burghausen (DE); List, Thomas, 84494 Neumarkt St. Veit (DE); Deinhammer, Wolfgang, Dr., 84489 Burghausen (DE); Heyen, Georges, Dr., 4053 Embourg (BE)
(74) Vertreter: Schuderer, Michael, Dr.

(56) Entgegenhaltungen:
- US-A- 2 395 800
- FRANK G YOUNG ET AL: "Conversion of ketone enol esters to beta-diketones by intramolecular thermal rearrangement and by intermolecular acylations using boron fluoride" JOURNAL OF THE AMERICAN CHEMICAL SOCIETY., Bd. 72, 1950, Seiten 3635-2642, XP002145724 AMERICAN CHEMICAL SOCIETY, WASHINGTON, DC., US ISSN: 0002-7863

## Beschreibung

Die Erfindung betrifft ein Verfahren zur Herstellung von 1,3-Diketonen durch thermische Umlagerung der entsprechenden isomeren Enolester, sowie eine Vorrichtung zur Durchführung des Verfahrens.

Journal of the American Chemical Society 72, (1950) 3635-3642 beschreibt die Umwandlung von keton-Enolethern zu β-Diketonen durch intramolekulare thermische Umlagerung und durch intermolekulare Acylierung mit Borfluorid.

Aus der US-A 2395600 ist bekannt, 1,3-Diketone durch thermische Umlagerung der entsprechenden isomeren Enolester bei einer Temperatur von 300°C bis 700°C in einem Rohrreaktor herzustellen, wobei das gasförmige Produktgemisch anschließend kondensiert wird und das Reinprodukt abdestilliert wird. Nachteilig ist, daß bei Reaktionen dieser Art Radikalmechanismen ablaufen, die neben unerwünschten Nebenprodukten auch zu Koksbildung führen und so eine Reinigung des Reaktors mit Produktionsausfall erforderlich wird. Diese Nebenprodukte reduzieren die Selektivität und müssen destillativ abgetrennt werden. In der DE-A 2047320 (US-A 3794686) wird eine Vorgehensweise beschrieben, bei der zur Verhinderung der Koksabscheidung Bleitetraalkyl dem Reaktionsgemisch zugegeben wird. Im Derwent-Abstract zur JP-A 63159337 wird beschrieben wie durch Zugabe von Wasser die Selektivität bei der Herstellung von Acetylaceton aus Isopropenylacetat verbessert und zugleich die Verkokungsgeschwindigkeit verringert wird. Nachteilig hierbei ist jedoch, daß das zugeführte Wasser einerseits zur Hydrolyse des Enolesters, andererseits zu Korrosionsproblemen führt.

Es bestand somit die Aufgabe, ein Verfahren zu entwickeln, mit dem auch ohne die Zugabe von Promotoren oder Wasser ein höherer Umsatz, bei zumindest gleichbleibender Selektivität, und geringere Koksbildung erhalten werden.

Gegenstand der Erfindung ist ein Verfahren zur Herstellung von 1,3-Diketonen mittels thermischer Umlagerung der entsprechenden isomeren Enolester in einem Rohrreaktor 1 bei einer Temperatur von 350°C bis 700°C, und anschließender Abkühlung und destillativer Aufarbeitung des Reaktionsproduktes, dadurch gekennzeichnet, daß der Rohrreaktor 1 nicht über die volle Länge, sondern nur im vorderen Abschnitt 1a, der sich über einen Bereich von mindestens 1/4 bis maximal 3/4 der gesamten Rohrlänge erstreckt, gerechnet vom Reaktoreingang aus, beheizt wird, und der verbleibende unbeheizte Abschnitt 1b des Rohrreaktors 1 thermisch isoliert wird.

Ein weiterer Gegenstand der Erfindung ist eine Vorrichtung zur Durchführung des Verfahrens umfassend einen Rohrreaktor 1 mit einem vorderen, nicht isolierten Rohrabschnitt 1a, welcher sich über einen Bereich von mindestens 1/4 bis maximal 3/4 der gesamten Rohrlänge erstreckt, gerechnet vom Reaktoreingang aus, und welcher mit einem oder mehreren Heizelementen 6 ausgestattet ist, und einem hinteren Rohrabschnitt 1b, welcher mit einer Kühlung oder Isolierung 2 ausgestattet ist, und mit einem Überleitungsrohr 3 mit einer Kolonne 4 verbunden ist, sowie mit einer Leitung 5 zur Zuleitung von Kühlflüssigkeit ausgestattet ist.

Die Umlagerung zu den 1,3-Diketonen erfolgt nach folgender Gleichung: CR¹R⁴ = CR²OCOR³ ----- > R²COCR¹R⁴COR³ (1), wobei für die als Edukte eingesetzten Enolester mit der allgemeinen Formel CR¹R⁴ = CR²OCOR³ und für die 1,3-Diketone mit der allgemeinen Formel R²COCR¹R⁴COR³ gilt, daß R¹,R²,R³ und R⁴ gleich oder verschieden sein können und die Bedeutung Wasserstoff, verzweigter oder unverzweigter Alkylrest mit 1 bis 8 C-Atomen oder Phenylrest haben können. Als Edukte bevorzugt werden Enolester bei denen R¹,R²,R³ und R⁴ gleich oder verschieden sind und die Bedeutung Wasserstoff oder Alkylrest mit 1 bis 3 C-Atomen haben. Besonders bevorzugt als Edukt wird Isopropenylacetat 1-Propen-2-ol-acetat) mit R¹ = R⁴ = H und R² = R³ = CH₃, welches zu Acetylaceton (2,4-Pentandion) umgelagert wird.

Vor der Umlagerung im Rohrreaktor wird das Edukt in einem Verdampfer 7, beispielsweise einem Umlaufverdampfer oder Spiralrohrverdampfer, bei einem Überdruck von 0.8 bar bis 1.5 bar, vorzugsweise bei Normaldruck, auf eine Temperatur von 90°C bis 110°C, vorzugsweise von 103°C bis 105°C erhitzt und verdampft. Das dampfförmige Edukt wird in den Rohrreaktor 1 geleitet.

Der Rohrreaktor 1 besteht aus einem korrosionsfesten Material, üblicherweise aus Edelstahl oder Quarzglas, bevorzugt wird Edelstahl. Der Rohrreaktor 1 kann als gerades Einzelrohr, als Spiralrohr oder als Rohrbündel ausgestaltet sein. Bevorzugt wird ein Einzelrohr, welches aus V-förmig angeordneten, gegebenenfalls konzentrisch ausgerichteten, Rohrschenkeln besteht. Die Dimensionierung der Reaktionsrohre wird vorzugsweise so gestaltet, daß im hinteren Rohrabschnitt 1b ein größeres Volumen/Oberflächen-Verhältnis als im vorderen Rohrabschnitt 1a resultiert. Besonders bevorzugt beträgt im vorderen Rohrabschnitt 1a das Volumen/Oberflächen-Verhältnis von 0.01 m bis 0.4 m, und im hinteren Rohrabschnitt 1b von 0.03 m bis 0.6 m.

Zur Beheizung ist der Rohrreaktor mit einem oder mehreren Heizelementen 6, beispielsweise einer Strahlungsheizung oder einer elektrischen Widerstandsheizung, vorzugsweise einer elektrischen Widerstandsheizung, ausgerüstet. Ist der Rohrreaktor 1 ein Einzelrohr mit V-förmig angeordneten Rohrschenkeln, so kann die Beheizung der Rohrschenkel einzeln erfolgen. Der während der Umlagerungsreaktion nicht beheizte Rohrabschnitt 1b kann gegebenenfalls auch mit einem oder mehreren Heizelementen 6 ausgestattet sein. Mittels dieser Ausführungsform kann gegebenenfalls vor dem Start der Umlagerungsreaktion das Rohr kurzzeitig über die gesamte Länge beheizt werden, um so der Kondensation des Edukts an der Rohrwand, mit damit einhergehender Verkrustung, in der Anfangsphase vorzubeugen.

Der nicht beheizte Abschnitt 1b des Rohrreaktors wird vorzugsweise mit einer thermischen Isolierung 2, beispielsweise einer Mineralwollmatte ausgerüstet, um eine möglichst adiabatische Reaktionsführung in diesem Bereich zu gewährleisten. Gegebenenfalls kann dieser Rohrabschnitt 1 b auch mit einem Kühlelement ausgestattet werden.

Die Umlagerungsreaktion wird bei Normaldruck und einer Temperatur von 350°C bis 700°C, vorzugsweise von 450°C bis 550°C durchgeführt. Der Energieeintrag zur Beheizung erfolgt dabei im vorderen Abschnitt 1a des Reaktionsrohres 1, der sich über einen Bereich von mindestens 1/4 bis maximal 3/4 der gesamten Rohrlänge, gerechnet ab Reaktoreingang, erstreckt. Bevorzugt erstreckt sich der beheizte Rohrabschnitt 1a über einen Bereich der mindestens die vordere Hälfte bis maximal 3/4 der gesamten Rohrlänge umfaßt. Besonders bevorzugt werden die vorderen zwei Drittel des Reaktionsrohres 1 beheizt. Der Energieeintrag zur Beheizung des entsprechenden Rohrabschnittes 1a kann gleichmäßig erfolgen. Möglich ist auch eine Vorgehensweise bei der im vorderen Teil des beheizten Rohrabschnittes 1a mehr Wärmeenergie eingetragen wird als im hinteren Teil. Dazu werden im vorderen Teil 50 % bis 150 % mehr Wärmeenergie eingetragen als im hinteren Teil des beheizten Rohrabschnitts 1a. Bevorzugt wird ein Verfahren bei dem mindestens das vordere Viertel bis maximal die vordere Hälfte des beheizten Rohrabschnitts 1a stärker beheizt wird als der hintere Teil.

Nach Erreichen der Umlagerungstemperatur im beheizten Teil des Reaktors 1 wird die Umlagerungsreaktion möglichst adiabatisch weitergeführt. Die Reaktion wird dabei vorzugsweise so geführt, daß bei Normaldruck die Temperatur T1 am Übergang vom beheizten Rohrabschnitt 1a zum unbeheizten Rohrabschnitt 1b im allgemeinen von 470°C bis 510°C, besonders bevorzugt 480°C bis 500°C beträgt. Bevorzugt werden Reaktionsbedingungen, bei denen die Temperatur T2 am Reaktorausgang höher ist als die Temperatur T1 am Übergang zwischen beheizter und unbeheizter Zone. Besonders bevorzugt sollte die Temperaturdifferenz zwischen T2 und T1 mindestens 20°C betragen.

In einer weiteren bevorzugten Ausführungsform wird die Reaktion so geführt, daß das Edukt in dem beheizten Rohrabschnitt 1a innerhalb von 0.5 s bis 1 s auf die Reaktionstemperatur gebracht wird, weitere 0.5 s bis 2 s in der unbeheizten Reaktionszone 1b auf diesem Temperaturniveau gehalten wird, und anschließend das Produktgemisch innerhalb von 0.1 s bis 1 s auf 100°C bis 200°C abgekühlt wird.

Im allgemeinen wird das gasförmige Edukt bzw. Reaktionsgemisch mit einer Raumgeschwindigkeit von 5 m/s bis 20 m/s durch den Reaktor geführt. Nach dem Austritt aus dem Rohrreaktor wird das Reaktionsgemisch abgekühlt. Die Kühlung kann mittels eines Wärmetauschers erfolgen, bevorzugt wird allerdings mittels Quenchen abgekühlt.

Die Kühlung erfolgt dabei in einem Überleitungsrohr 3 zwischen dem Rohrreaktor 1 und der Kolonne 4 zur Auftrennung des Produktgemisches. Das aus dem Reaktor austretende gasförmige Produktgemisch wird in der bevorzugten Ausführungsform durch Einspritzen einer Kühlflüssigkeit über Leitung 5 in das Überleitungsrohr 3, möglichst unmittelbar nach dem Reaktorausgang, üblicherweise in einem Abstand von bis zu 2 m, vorzugsweise in einem Abstand von 1 m bis 2 m, auf eine Temperatur von 100°C bis 200°C, vorzugsweise 110°C bis 130°C abgekühlt. Die bei diesem Temperaturniveau nicht kondensierbaren gasförmigen Anteile, wie CO oder CO₂, werden über Leitung 10 abgezogen.

Als Kühlflüssigkeit geeignet sind beispielsweise Wasser und das jeweilige Reaktionsprodukt (1,3-Diketon), letzteres beispielsweise in Form des Sumpfproduktes der Kolonne 4. Bevorzugt wird das Sumpfprodukt der Kolonne 4 als Kühlflüssigkeit eingesetzt.

Die Temperatur der Kühlflüssigkeit beträgt im allgemeinen 25°C bis 130°C, je nach dem welche Flüssigkeit als Kühlflüssigkeit eingesetzt wird. Bevorzugt wird ein Temperaturbereich von 50°C bis 80°C. Wird in der bevorzugten Ausführungsform der Kolonnensumpf aus der Produktauftrennung als Kühlflüssigkeit verwendet, wird dieser gegebenenfalls vorher auf die genannte Temperatur abgekühlt. Im allgemeinen beträgt das Gewichtsverhältnis von Produktstrom zu Quenchflüssigkeit 1 : 2 bis 1 : 5. Mit dieser Vorgehensweise bleibt der Produktstrom auf einem Temperaturniveau von im allgemeinen 125°C bis 130°C, bei dem sichergestellt wird, daß bei der anschließenden Aufarbeitung des Produktstromes in der Kolonne 4 nicht umgesetztes Edukt, ohne weiteren Energieaufwand, über Kopf über Leitung 8, oder über eine separate Leitung 11, abgetrennt werden kann.

Nach der Quenchung wird der abgekühlte Produktstrom der Kolonne 4 zur Auftrennung des Produktgemisches zugeführt. Über den Kolonnenkopf werden dabei in an sich bekannter Weise leichtsiedendere Bestandteile über Leitung 8, und nicht umgesetztes Edukt über Leitung 11, entfernt, während das gebildete 1,3-Diketon im Kolonnensumpf anfällt und über Leitung 9 weiteren Reinigungsschritten zur Abtrennung von Nebenprodukten zugeführt werden kann.

Mit dem erfindungsgemäßen Verfahren wird überraschenderweise ohne den Zusatz von Promotoren oder Wasser eine deutliche Steigerung der Ausbeute und der Selektivität, sowie eine drastische Minimierung der Verkokung erreicht. Wie der Vergleich des nachfolgenden Beispiels 1 mit dem Vergleichsbeispiel 2 zeigt, wird die Ausbeute um 16 % und die Selektivität um 23 % gesteigert. Das Wartungsintervall zur Reinigung von Koksablagerungen wird von 3 Wochen auf 12 Monate um das 16-fache verlängert. Der Energieeintrag pro Mengeneinheit des Zielproduktes ist gegenüber dem Stand der Technik deutlich reduziert, da die bei der Quenche anfallende Reaktionswärme zur Abtrennung der Leichtsieder genutzt werden kann.

Das Verfahren wird im folgenden beispielhaft an der Umlagerung von 1-Propen-2-ol-acetat (Isopropenylacetat) zu Acetylaceton (2,4-Pentandion) beschrieben, gilt aber sinngemäß auch für die in Gleichung (1) genannten Stoffe.

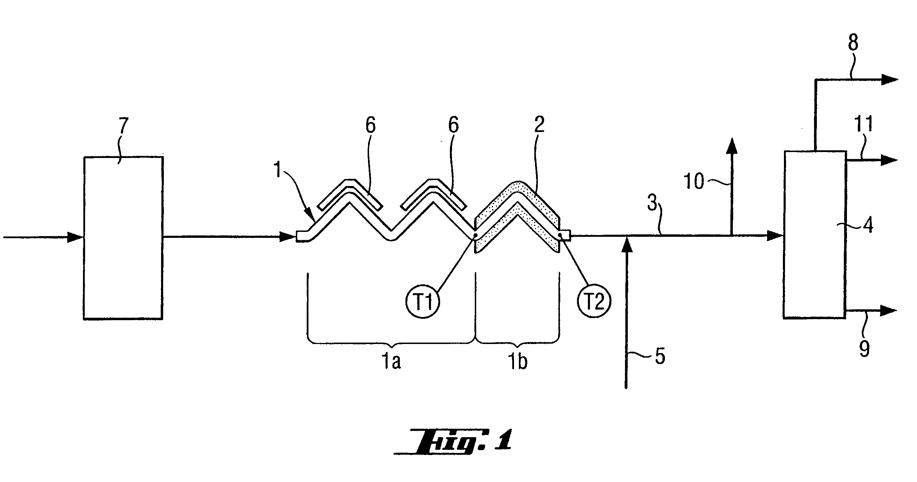

Die Vorrichtung wird in Figur 1 näher erläutert, welche eine bevorzugte Ausführungsform der Erfindung zeigt.

### Beispiel 1:

500 kg/h Isopropenylacetat wurden über einen Umlaufverdampfer 7, bei Normaldruck in einen widerstandsbeheizten Rohrreaktor 1 geleitet, welcher aus sechs V-förmig, konzentrisch angeordneten Rohrschenkeln mit jeweils 4.40 m Länge bestand, wobei die Rohre in den vorderen zwei Dritteln des Rohrreaktors einen Durchmesser von 0.08 m (V/O = 0.02 m) und im hinteren Drittel einen Durchmesser von 0.2 m (V/O = 0.05) hatten. Der Rohrreaktor wurde in den vorderen zwei Dritteln beheizt und im hinteren Drittel mit Mineralwollmatte isoliert. Der Wärmeenergieeintrag wurde so gesteuert, daß die Heizleistung in der vorderen Hälfte (erstes Drittel des Rohrreaktors 1) des beheizten Abschnittes 1a doppelt so hoch war, wie in der hinteren Hälfte (zweites Drittel des Rohrreaktors 1). Die Isolierung in der unbeheizten Zone 1b wurde so gewählt, daß die Abstrahlverluste so gering waren, daß sich zwischen den Meßpunkten T1 am Ende der beheizten Zone und T2 am Reaktorausgang eine Temperaturdifferenz ΔT von 20°C einstellte, wobei T2 > T1 war.
Das mit einer Temperatur von 530°C austretende Produktgemisch wurde unmittelbar nach dem Reaktorausgang durch Einleiten eines Quenchstromes in das Überleitungsrohr 3 auf 128°C abgekühlt,
wobei als Quenchflüssigkeit das Sumpfprodukt der Kolonne 4 eingesetzt wurde. Flüchtige, nicht kondensierte Produktbestandteile wurden über Leitung 10 entfernt und der abgekühlte Produktstrom zur weiteren Aufarbeitung der Kolonne 4 zugeführt. Dieses Verfahren ergab die in Tabelle 1 zusammengestellten Leistungscharakteristika.

### Vergleichsbeispiel 2:

Wie in Beispiel 1 wurden 500 kg/h Isopropenylacetat über einen Umlaufverdampfer 7 bei Normaldruck in den widerstandsbeheizten Rohrreaktor 1 geleitet. Im Unterschied zur Vorgehensweise in Beispiel 1 wurde das Reaktionsrohr 1 über die volle Länge gleichmäßig beheizt. Die Isolierung des Reaktionsrohres nach der Aufheizzone wurde entfernt. Nach kurzer Zeit stellt sich an T1 und T2 eine Temperaturdifferenz ΔT von 20°C ein, wobei T2 < T1 war. Der Produktstrom wurde analog Beispiel 1 mit einem Produktstrom-Quenchstrom-Verhältnis von 1 : 1 abgekühlt. Die mit der beschriebenen Reaktionsführung erreichten Leistungscharakteristika sind in Tabelle 1 zusammengestellt.

**Tabelle 1**

| | Beispiel 1 | Vergl.bsp. 2 |
|---|---|---|
| Feed Isopropenylacetat | 500 kg/h | 500 kg/h |
| Energieeintrag gesamt | 108 KW | 108 KW |
| Energieverteilung in den einzelnen Dritteln des Reaktionsrohres | 2:1:0 | 1:1:1 |
| Raum-Zeit-Ausbeute Acetylaceton | 898 g/lxh | 777 g/lxh |
| Ausbeute Acetylaceton bezogen auf eingesetztes Isopropenylacetat (Umsatz) | 65.4 % | 56.6 % |
| Ausbeute Acetylaceton bezogen auf umgesetztes Isopropenylacetat (Selektivität) | 85.2 % | 69.1 % |
| Verhältnis Produktstrom:Quenchstrom | 1 : 3 | 1 : 1 |
| Reinigungsintervall | 12 Monate | 3 Wochen |

## Patentansprüche

1. Verfahren zur Herstellung von 1,3-Diketonen mittels thermischer Umlagerung der entsprechenden isomeren Enolester in einem Rohrreaktor 1 bei einer Temperatur von 350°C bis 700°C, und anschließender Abkühlung und destillativer Aufarbeitung des Reaktionsproduktes, **dadurch gekennzeichnet, daß** der Rohrreaktor 1 nicht über die volle Länge, sondern nur im vorderen Abschnitt 1a, der sich über einen Bereich von mindestens 1/4 bis maximal 3/4 der gesamten Rohrlänge erstreckt, gerechnet vom Reaktoreingang aus, beheizt wird, und der verbleibende unbeheizte Abschnitt 1b des Rohrreaktors 1 thermisch isoliert wird.

2. Verfahren nach Anspruch 1, **dadurch gekennzeichnet, daß** Enolester der Formel CR¹R⁴ = CR²OCOR³ eingesetzt werden, wobei R¹,R²,R³ und R⁴ gleich oder verschieden sein können und die Bedeutung Wasserstoff, verzweigter oder unverzweigter Alkylrest mit 1 bis 8 C-Atomen oder Phenylrest haben können.

3. Verfahren nach Anspruch 1 oder 2, **dadurch gekennzeichnet, daß** Isopropenylacetat zu Acetylaceton umgelagert wird.

4. Verfahren nach Anspruch 1 bis 3, **dadurch gekennzeichnet, daß** im vorderen Teil des beheizten Rohrabschnittes 1a mehr Wärmeenergie eingetragen wird als im hinteren Teil.

5. Verfahren nach Anspruch 1 bis 4, **dadurch gekennzeichnet, daß** bei Normaldruck die Temperatur T1 am Übergang vom beheizten Rohrabschnitt 1a zum unbeheizten Rohrabschnitt 1b von 470°C bis 510°C beträgt.

6. Verfahren nach Anspruch 1 bis 5, **dadurch gekennzeichnet, daß** die Temperatur T2 am Reaktorausgang höher ist als die Temperatur T1 am Übergang zwischen beheizter und unbeheizter Zone.

7. Verfahren nach Anspruch 1 bis 6, **dadurch gekennzeichnet, daß** das aus dem Reaktor austretende gasförmige Produktgemisch durch Einspritzen einer Kühlflüssigkeit, möglichst unmittelbar nach dem Reaktorausgang, auf eine Temperatur von 100°C bis 200°C abgekühlt wird.

8. Verfahren nach Anspruch 7, **dadurch gekennzeichnet, daß** als Kühlflüssigkeit Wasser oder das jeweilige Reaktionsprodukt eingesetzt wird.

9. Verfahren nach Anspruch 1 bis 8, **dadurch gekennzeichnet, daß** das Verhältnis von Produktstrom zu Quenchflüssigkeit von 1 : 1 2 bis 1 : 1 5 beträgt.

10. Verfahren nach Anspruch 1 bis 9, **dadurch gekennzeichnet, daß** die Reaktion so geführt, daß das Edukt in dem beheizten Rohrabschnitt 1a innerhalb von 0.5 s bis 1 s auf die Reaktionstemperatur gebracht wird, weitere 0.5 s bis 2 s in der unbeheizten Reaktionszone 1b auf diesem Temperaturniveau gehalten wird, und anschließend das Produktgemisch innerhalb von 0.1 bis 1 s auf 100°C bis 200°C abgekühlt wird.

11. Verfahren nach Anspruch 1 bis 10, **dadurch gekennzeichnet, daß** der Produktstrom auf ein Temperaturniveau von 125°C bis 130°C abgekühlt wird.

12. Vorrichtung zur Durchführung des Verfahrens nach Anspruch 1 bis 11 umfassend einen Rohrreaktor 1 mit einem vorderen, nicht isolierten Rohrabschnitt 1a, welcher sich über einen Bereich von mindestens 1/4 bis maximal 3/4 der gesamten Rohrlänge erstreckt, gerechnet vom Reaktoreingang aus, und welcher mit einem oder mehreren Heizelementen 6 ausgestattet ist, und einem hinteren Rohrabschnitt 1b, welcher mit einer Kühlung oder Isolierung 2 ausgestattet ist, und mit einem Überleitungsrohr 3 mit einer Kolonne 4 verbunden ist, sowie mit einer Leitung 5 zur Zuleitung von Kühlflüssigkeit ausgestattet ist.

13. Vorrichtung nach Anspruch 12, **dadurch gekennzeichnet, daß** im hinteren Rohrabschnitt 1b ein größeres Volumen/Oberflächen-Verhältnis als im vorderen Rohrabschnitt 1a resultiert.

14. Vorrichtung nach Anspruch 13, **dadurch gekennzeichnet, daß** im vorderen Rohrabschnitt 1a das Volumen/OberflächenVerhältnis von 0.01 m bis 0.4 m, und im hinteren Rohrabschnitt 1b von 0.03 m bis 0.6 m beträgt.

## Claims

1. Process for preparing 1,3-diketones by means of thermal rearrangement of the corresponding isomeric enol esters in a tube reactor 1 at a temperature of from 350°C to 700°C and subsequent cooling and work-up of the reaction product by distillation, **characterized in that** the tube reactor 1 is not heated over its full length but only in a front section 1a which extends over a region from at least % to at most ¾ of the total tube length, calculated from the reactor inlet, and the remaining unheated section 1b of the tube reactor 1 is thermally insulated.

2. Process according to Claim 1, **characterized in that** the enol esters used have the formula CR¹R⁴ = CR²OCOR³, where R¹, R², R³ and R⁴ may be identical or different and may each be hydrogen, a branched or unbranched alkyl radical having from 1 to 8 carbon atoms or a phenyl radical.

3. Process according to Claim 1 or 2, **characterized in that** isopropenyl acetate is rearranged to form acetylacetone.

4. Process according to any of Claims 1 to 3, **characterized in that** more thermal energy is introduced in the front part of the heated tube section 1a than in the downstream part.

5. Process according to any of Claims 1 to 4, **characterized in that**, at atmospheric pressure, the temperature T1 at the transition from the heated tube section 1a to the unheated tube section 1b is from 470°C to 510°C.

6. Process according to any of Claims 1 to 5, **characterized in that** the temperature T2 at the reactor outlet is higher than the temperature T1 at the transition between the heated and unheated zones.

7. Process according to any of Claims 1 to 6, **characterized in that** the gaseous product mixture leaving the reactor is cooled to a temperature of from 100°C to 200°C by spraying in a cooling liquid immediately downstream of the reactor outlet.

8. Process according to Claim 7, **characterized in that** the cooling liquid used is water or the respective reaction product.

9. Process according to any of Claims 1 to 8, **characterized in that** the ratio of product stream to quenching liquid is from 1:2 to 1:5.

10. Process according to any of Claims 1 to 9, **characterized in that** the reaction is carried out so that the starting material is brought to the reaction temperature in the heated tube section 1a within from 0.5 s to 1 s, is held at this temperature level in the unheated reaction zone 1b for a further 0.5-2 s, and the product mixture is subsequently cooled to from 100°C to 200°C within from 0.1 s to 1 s.

11. Process according to any of Claims 1 to 10, **characterized in that** the product stream is cooled to a temperature level of from 125°C to 130°C.

12. Apparatus for carrying out the process according to any of Claims 1 to 11 comprising a tube reactor 1 having a front, uninsulated tube section 1a which extends over a region from at least ¼ to at most ¾ of the total tube length, calculated from the reactor inlet, and is provided with one or more heating elements 6, and a downstream tube section 1b which is provided with cooling or insulation 2 and is connected via a transfer tube 3 to a column 4 and is also provided with a line 5 for the introduction of cooling liquid.

13. Apparatus according to Claim 12, **characterized in that** the downstream tube section 1b has a larger volume/surface area ratio than the front tube section 1a.

14. Apparatus according to Claim 13, **characterized in that** the volume/surface area ratio in the front tube section 1a is from 0.01 m to 0.4 m and that in the downstream tube section 1b is from 0.03 m to 0.6 m.

## Revendications

1. Procédé pour la préparation de 1,3-dicétones par transposition thermique des énolesters isomères correspondants, dans un réacteur tubulaire (1), à une température de 350°C à 700°C, et refroidissement subséquent et traitement final par distillation du produit de réaction, **caractérisé en ce que** le réacteur tubulaire (1) n'est pas chauffé sur toute sa longueur mais seulement dans le segment avant (1a) qui s'étend sur une zone d'au moins ¼ à ¾ au maximum de la longueur totale du tube, calculé à partir de l'entrée du réacteur, et le segment restant non chauffé (1b) du réacteur tubulaire (1) est isolé thermiquement.

2. Procédé selon la revendication 1, **caractérisé en ce qu'**on utilise des énolesters de formule CR¹R⁴=CR²OCOR³, dans laquelle R¹, R², R³ et R⁴ peuvent être identiques ou différents et peuvent représenter un atome d'hydrogène, un radical alkyle ramifié ou non ramifié ayant de 1 à 8 atomes de carbone ou le radical phényle.

3. Procédé selon la revendication 1 ou 2, **caractérisé en ce que** l'acétate d'isopropényle est transposé en acétylacétone.

4. Procédé selon l'une quelconque des revendications 1 à 3, **caractérisé en ce qu'**une plus grande quantité d'énergie thermique est introduite dans la partie avant du segment chauffé (la) du tube que dans la partie arrière.

5. Procédé selon l'une quelconque des revendications 1 à 4, **caractérisé en ce que** sous la pression normale la température T1 au niveau du passage du segment chauffé (1a) du tube au segment non chauffé (1b) du tube est de 470°C à 510°C.

6. Procédé selon l'une quelconque des revendications 1 à 5, **caractérisé en ce que** la température T2 à la sortie du réacteur est supérieure à la température T1 au niveau du passage entre zone chauffée et zone non chauffée.

7. Procédé selon l'une quelconque des revendications 1 à 6, **caractérisé en ce que** le mélange gazeux constituant le produit, sortant du réacteur, est refroidi par injection d'un liquide de refroidissement, le plus immédiatement possible après la sortie du réacteur, jusqu'à une température de 100°C à 200°C.

8. Procédé selon la revendication 7, **caractérisé en ce qu'**on utilise comme liquide de refroidissement l'eau ou le produit de réaction respectif.

9. Procédé selon l'une quelconque des revendications 1 à 8, **caractérisé en ce que** le rapport du courant de produit au liquide de refroidissement va de 1:2 à 1:5.

10. Procédé selon l'une quelconque des revendications 1 à 9, **caractérisé en ce que** la réaction est conduite de telle façon que le produit de départ est porté à la température de réaction, dans le segment chauffé (1a) du tube en l'espace de 0,5 seconde à 1 seconde, est maintenu à ce niveau de température pendant encore 0,5 s à 2 s dans la zone de réaction non chauffée (1b), et ensuite le mélange constituant le produit est refroidi jusqu'à 100°C à 200°C en l'espace de 0,1 s à 1 s.

11. Procédé selon l'une quelconque des revendications 1 à 10, **caractérisé en ce que** le courant de produit est refroidi jusqu'à un niveau de température de 125°C à 130°C.

12. Dispositif pour la mise en oeuvre du procédé selon l'une quelconque des revendications 1 à 11, comprenant un réacteur tubulaire (1) comportant un segment tubulaire avant (1a) non isolé, qui s'étend sur une zone d'au moins ¼ à ¾ au maximum de la longueur totale du tube, calculé à partir de l'entrée du réacteur, qui est muni d'un ou de plusieurs éléments chauffants (6), et un segment tubulaire arrière (1b) qui est muni d'un refroidissement ou d'une isolation (2) et est raccordé à une colonne (4) par un conduit de transfert (3), et est également muni d'un conduit (5) pour l'arrivée du liquide de refroidissement.

13. Dispositif selon la revendication 12, **caractérisé en ce que** dans le segment arrière (1b) du tube il résulte un rapport volume/surface plus élevé que dans le segment avant (1a) du tube.

14. Dispositif selon la revendication 13, **caractérisé en ce que** le rapport volume/surface est de 0,01 m à 0,4 m dans le segment avant (1a) du tube et de 0,03 m à 0,6 m dans le segment (1b) du tube.
